(19) Europäisches Patentamt
European Patent Office
Office européen des brevets

(11) Numéro de publication: 0 179 676 B1

(12) **FASCICULE DE BREVET EUROPEEN**

(45) Date de publication du fascicule du brevet: **11.05.88**

(51) Int. Cl.⁴: **C 25 B 3/02**

(21) Numéro de dépôt: **85401651.6**

(22) Date de dépôt: **16.08.85**

(54) **Procédé de préparation d'acide glyoxylique par oxydation électrochimique anodique du glyoxal.**

(30) Priorité: **04.09.84 FR 8413607**
**28.11.84 FR 8418117**

(43) Date de publication de la demande:
**30.04.86 Bulletin 86/18**

(45) Mention de la délivrance du brevet:
**11.05.88 Bulletin 88/19**

(84) Etats contractants désignés:
**AT BE CH DE FR GB IT LI LU NL SE**

(56) Documents cités:
**FR - A - 2 443 517**

**JOURNAL OF THE ELECTROCHEMICAL SOCIETY: ELECTROCHEMICAL SCIENCE AND TECHNOLOGY, vol. 130, no. 6, juin 1983, pages 1305-1312, Manchester, New Hampshire, US; E.P.M. LEIVA et al.: "The influence of platinum electrode surface on the electroadsorption and electro-oxidation of methanol in acid solutions"**
**CHEMICAL ABSTRACTS, vol. 90, no. 24, 11 juin 1979, page 515, no. 194539j, Columbus, Ohio, US; G. INZELT et al.: "Electrochemical behavior of ethylene glycol and its oxidation products on a platinum electrode. III. Effect of chemisorption on the kinetics of oxidation of glyoxal and glyoxalic acid", ACTA CHIM. ACAD. SCI. HUNG. 1978 (Pub. 1979). 98(4), 403-19**

(73) Titulaire: **SOCIETE FRANCAISE HOECHST Société anonyme dite:, 3, avenue du Général de Gaulle, F-92800 Puteaux (FR)**

(72) Inventeur: **Pierre, Gérard, 34 rue Chamechaude, F-38320 Eybans (FR)**
Inventeur: **El Kordi, Mokhlis, 8, Meet Masoud, Sheben el Koom El Monofia (EG)**
Inventeur: **Cauquis, Georges, 6 bis avenue Jean Perrot, F-38000 Grenoble (FR)**

(74) Mandataire: **Rinuy, Santarelli, 14, avenue de la Grande Armée, F-75017 Paris (FR)**

**EP 0 179 676 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de préparation d'acide glyoxylique par oxydation électrochimique anodique du glyoxal.

Il est connu d'oxyder, en milieu aqueux, le glyoxal en acide glyoxylique soit par de l'acide nitrique (brevet français N° 1 326 605 et 2 372 141), soit par voie électrochimique (demande de brevet français N° 2 443 517).

Plus généralement, on sait que certains procédés électrochimiques permettent d'accéder à l'acide glyoxylique tels que la réduction cathodique de l'acide oxalique (brevet français N° 2 151 150), l'oxydation anodique de l'éthylèneglycol ou de l'acide tartrique (voir, par exemple, les travaux de G. HORANYI et al. Acta Chim. Acad. Sci. Hung., 1978, 98, 49-66, 367-373, Magyar Kém. Folyoirat, 1978, 61-68, 217-225, 255-362, 469-475).

Toutefois, ces procédés connus présentent des inconvénients. Ainsi, (a) l'oxydation nitrique du glyoxal demande des investissements importants tant pour isoler l'acide glyoxylique que pour dénitrifier les eaux de lavage, (b) l'acide oxalique et l'acide tartrique sont des matières premières peu économiques, (c) l'oxydation anodique de l'éthylèneglycol est peu performante et (d) le procédé d'oxydation anodique du glyoxal nécessite l'emploi d'électrolyseurs équipés de membranes échangeuses d'ions.

Or, la Demanderesse a découvert avec étonnement un procédé d'oxydation électrochimique anodique du glyoxal permettant de préparer avec de bons rendements et une bonne sélectivité de l'acide glyoxylique tout en se libérant des inconvénients des procédés connus. Le procédé selon la présente invention consiste à soumettre, sous agitation, à une température comprise entre 0°C et 70°C, une solution aqueuse de glyoxal à une oxydation électrochimique anodique dans un électrolyseur comportant, au moins un compartiment cathodique contenant une cathode et un catholyte, au moins un compartiment anodique contenant une anode et un anolyte constitué par une solution aqueuse de glyoxal et un électrolyte et, entre ces deux compartiments, au moins un séparateur, ledit procédé étant caractérisé en ce que l'anode est dopée par de faibles quantités d'adatomes métalliques choisis parmi l'argent, le bismuth, le cuivre, l'étain, le thallium.

Suivant une autre caractéristique, le séparateur est avantageusement en verre fritté. De préférence un tel séparateur en verre fritté présentera un diamètre moyen de pores compris entre 5 et 15 micromètres.

Suivant un autre mode de réalisation avantageux, le séparateur est en une membrane échangeuse d'anions contrairement aux membranes échangeuses de cations déjà utilisées dans des procédés connus d'oxydation électrochimique du glyoxal (voir par exemple le brevet français N° 2 443 517). Une telle membrane échangeuse d'anions permet, d'une part, d'éviter la migration vers la cathode des cations utilisés pour doper l'anode et, d'autre part, de maintenir constante la concentration en ions chlorure dans le compartiment anodique.

Par ailleurs, cette membrane anionique favorise la consommation de glyoxal, augmente les rendements en acide glyoxylique et fournit un acide glyoxylique pauvre en glyoxal résiduel.

Comme exemple de membrane anionique pouvant être utilisée conformément à l'invention, on peut citer les membranes anioniques d'origine et de nature très diverses comme les membranes anioniques à groupement ammonium quaternaires sur squelette polyvinylique, polystyrène, polyvinyllidène fluoré, texturées ou non.

De façon plus particulière, on citera les membranes commerciales RAIPORE 5035 L ou IONAC 3475 vendues respectivement par les sociétés:

— RAI Research Corporation, 225 Marcus Boulevard Hauppauge, L.I. N.Y. 11788, Etats-Unis d'Amérique,

— IONAC Chemical Company, Birmingham, N.J. - Etats-Unis d'Amérique.

Le rôle exact joué par ces adatomes n'est pas connu mais la Demanderesse a constaté avec étonnement qu'ils favorisaient l'oxydation du glyoxal et qu'ils permettaient d'obtenir une solution aqueuse d'acide glyoxylique pauvre en glyoxal non transformé.

L'anode est dopée par ces adatomes par dépôt électrolytique soit préalablement, soit au cours du procédé selon l'invention, à partir d'un de leurs dérivés hydrosolubles ou légèrement hydrosolubles tels que le nitrate d'argent, l'oxyde de bismuth III, le chlorure cuivreux, le chlorure stanneux, le nitrate de thallium III. Selon l'invention, l'anode peut être dopée avec des adatomes de nature identique ou différente.

Les adatomes préférés pour doper l'anode sont l'argent et l'étain.

Le dépôt préalable est effectué par un procédé classique connu en soi. Généralement, on l'effectue dans un électrolyseur à trois électrodes: une électrode de référence (à calomel saturée en KCl, par exemple), une électrode auxiliaire en platine et l'électrode à doper dont l'électrolyte contient en solution un ou plusieurs dérivés hydrosolubles du ou des adatomes choisis à une concentration de $1.10^{-4}$ à $1.10^{-2}$ molaire, plus on dépose électrochimiquement le ou les métaux choisis sur l'électrode à doper en utilisant environ 20 + 10 coulombs par $cm^2$ d'électrode. Un tel dépot peut aussi être réalisé en introduisant dans le compartiment anodique au début du procédé un ou plusieurs dérivés hydrosolubles du ou des adatomes choisis à une concentration de $1.10^{-4}$ à $1.10^{-2}$ molaire.

Le procédé selon l'invention est mis en œuvre à une température comprise entre 0 et 70°C, de préférence entre 20 et 70°C, et avantageusement à 50°C.

La cathode de l'électrolyseur est constituée par un matériau conducteur de l'électricité, stable chimiquement et électrochimiquement dans le catholyte sous les conditions opératoires considérées. Un tel matériau est notamment, le platine, l'acier inoxydable.

L'anode est en platine ou en carbone vitreux. L'anolyte contient essentiellement de l'eau, du glyoxal, un électrolyte et éventuellement, à faibles concentrations, un ou plusieurs dérivés hydrosolubles du ou des adatomes choisis pour doper l'anode.

L'électrolyte habituellement utilisé est un produit minéral très soluble dans l'eau contenant un anion choisi dans le groupe constitué par les anions chlorure, nitrite, nitrate et sulfate. Avantageusement, l'électrolyte contient des ions chlorure apportés de préférence par du chlorure d'hydrogène.

Le catholyte contient essentiellement de l'eau et un électrolyte, généralement, qualitativement et quantitativement identique à celui présent dans l'anolyte. Toutefois, il peut en être différent et contenir alors des ions hydroxyde.

Les concentrations molaires du glyoxal dans l'anolyte peuvent varier dans de larges limites mais, habituellement, elles sont comprises entre 0,2 et 5 M, avantageusement, elles sont comprises entre 0,5 et 2 M.

Au fur et à mesure de l'avancement de la réaction d'oxydation anodique, l'anolyte s'appauvrit en glyoxal et s'enrichit en acide glyoxylique selon l'équation (1):

$$\text{OHC-CHO} + H_2O - 2e^- \rightarrow \text{OHC-COOH} + 2\,H^+ \quad (1)$$

Au vu de cette équation (1), il est nécessaire d'utiliser 193 000 coulombs pour oxyder une mole de glyoxal et cette valeur est désignée Qth.

L'acide glyoxylique formé peut également être oxydé électrochimiquement en acide oxalique selon l'équation (2):

$$\text{OHC-COOH} + H_2O - 2e^- \rightarrow \text{HOOC-COOH} + 2H^+ \quad (2)$$

et enfin l'acide oxalique peut être dégradé par oxydation décarboxylante selon l'équation (3):

$$\text{HOOC-COOH} + H_2O - 2e^- \rightarrow 2\,CO^2 + H_2O + 2H^+ \quad (3).$$

L'oxydation du glyoxal en acide glyoxylique est donc en compétition d'une part avec l'oxydation de l'acide glyoxylique formé en acide oxalique et d'autre part, avec la dégradation oxydante décarboxylante de l'acide oxalique éventuellement présent.

En fin de réaction, l'anolyte contient essentiellement de l'eau, de l'acide glyoxylique, du glyoxal non transformé, des électrolytes et éventuellement de l'acide oxalique. La quantité de glyoxal résiduel est faible et l'emploi d'une anode dopée selon l'invention permet d'obtenir en fin de réaction un anolyte ne contenant que de faibles quantités de glyoxal résiduel.

La concentration en acide oxalique est d'autant plus importante que le rapport:

$$\rho = \frac{Q}{Qth}$$

(où Q = quantité d'électricité consommée et Qth = quantité d'électricité théorique)
est plus élevé.

La densité d'électricité à la cathode est généralement comprise entre 0,1 et 10 A par $dm^2$, de préférence entre 0,5 et 5 A par $dm^2$ et avantageusement elle est de 1 A par $dm^2$.

La différence de potentiel appliquée aux électrodes, ci-après désignée V, est fonction de la résistivité électrique des solutions contenues dans les divers compartiments et de leurs concentrations en électrolyte.

Généralement, une différence de potentiel comprise entre 2 et 10 volts est suffisante pour maintenir la densité électrique imposée aux électrodes. Habituellement, on obtient un anolyte et un catholyte présentant une résistivité électrique satisfaisante en utilisant un électrolyte tel que défini précédemment à une concentration exprimée en équivalent grammelitre comprise entre 0,26 et 3. Un tel électrolyte est notamment le chlorure d'hydrogène, le nitrite de potassium, le nitrate de potassium, le chlorure de sodium, le sulfate de sodium.

L'électrolyte préféré est le chlorure d'hydrogène à la concentration molaire de 1 ± 0,3.

Une variante du procédé selon l'invention consiste à utiliser un catholyte aqueux présentant un pH supérieur à 7, constitué essentiellement par de l'eau et un hydroxyde de métal alcalin.

L'hydroxyde de métal alcalin est de l'hydroxyde de sodium ou de l'hydroxyde de potassium, de préférence de l'hydroxyde de sodium à une concentration molaire de 1.

Cette variante du procédé selon l'invention peut être réalisée soit dans un électrolyseur classique à deux compartiments séparés par un diaphragme en verre fritté, soit avantageusement dans un électrolyseur à trois compartiments en série, séparés par un diaphragme en verre fritté: un compartiment anodique contenant l'anode et l'anolyte, un compartiment cathodique contenant la cathode et le catholyte et un compartiment central intermédiaire contenant une solution identique au catholyte utilisé.

On notera avec intérêt que la diffusion des différentes espèces chimiques présentes dans chaque compartiment est faible.

Les exemples donnés ci-après illustrent l'invention sans toutefois la limiter.

Les concentrations en glyoxal, acide glyoxylique et acide oxylique sont exprimées, sauf indication contraire, en millimoles par litres de solution.

Le rendement chimique, Rc, et le rendement électrique, Re, sont calculés par les équations suivantes:

$$\text{Rendement chimique} = \frac{\text{Nombre de moles d'acides glyoxylique obtenu}}{\text{Nombre de moles de glyoxal disparu}} \times 100$$

$$\text{Rendement électrique} = \frac{\text{Quantité d'électricité théorique pour obtenir l'acide glyoxylique dosé}}{\text{Quantité d'électricité utilisée}} \times 100$$

L'électrolyseur utilisé dans les exemples 1 à 13 présente deux compartiments séparés par un diaphragme en verre fritté de diamètre moyen des pores de 10 + 5 micromètres:

— un compartiment cathodique de 80 $cm^3$ équipé d'une cathode en platine,

— un compartiment anodique de 120 $cm^3$ équipé d'une anode de surface utile de 15 $cm^2$.

L'électrolyseur utilisé dans les autres exemples, 14 à 16, présente trois compartiments en série:

— un compartiment cathodique de 80 $cm^3$ équipé d'une cathode en platine,

— un compartiment central de 30 $cm^3$ séparé des compartiments anodique et cathodique par un diaphragme en verre fritté de diamètre moyen des pores de 10 + 5 micromètres,

— un compartiment anodique de 120 $cm^3$ équipé d'une agitation magnétique et d'une anode de surface utile de 15 $cm^2$.

TABLEAU I - EXEMPLES 1-13 - Influence des adatomes déposés sur anode de platine

*Conditions opératoires:* Electrodes: platine
I: 1 $A/dm^2$
V: 2,8 V

Catholyte: HCl 1,1 N
Anolyte: HCl 1,1 N
glyoxal 0,88 M

| | Exemples 1/2 | | Exemples 3/4 | | Exemples 5 à 7 | | | Exemples 8/9 | | Exemples 10/11 | | Exemples 12/13 | |
|---|---|---|---|---|---|---|---|---|---|---|---|---|---|
| Adatome | Néant | | Sn | | Ag | | | Cu | | Tl | | Bi | |
| Nature | | | $SnCl_2$ | | $AgNo_3$ | | | CuCl | | $Tl(NO_3)_3$ | | $Bi_2O_3$ | |
| Quantité mmol/l | | | 1,2 | | 1,35 | | | 2,3 | | 0,6 | | 0,5 | |
| Température °C | 20 | 50 | 20 | 50 | 20 | 50 | 50 | 20[1] | 50[2] | 20 | 50 | 50 | 50[3] |
| Q/Qth | 1,2 | 1,2 | 1,23 | 1,2 | 1,01 | 1,2 | 1,65 | 1,28 | 1,2 | 1,15 | 1,27 | 1,22 | 1,25 |
| *Résultats* | | | | | | | | | | | | | |
| Glyoxal résiduel | 151 | 99 | 60 | 17 | 90 | 35 | 7 | 120 | 51 | 120 | 26 | 65 | 52 |
| Acide glyoxylique | 463 | 482 | 446 | 554 | 500 | 613 | 473 | 594 | 1200 | 500 | 540 | 513 | 540 |
| Acide oxalique | 38 | 8,5 | 77 | 64 | 23 | 77 | 55 | 44 | 200 | 44 | 111 | 66 | 77 |
| Rendement chimique | 63,5 | 62 | 55 | 63 | 63 | 72,5 | 54 | 78 | 72 | 66 | 63 | 63 | 65 |
| Rendement électrique | 44 | 46 | 41 | 52 | 57 | 59 | 31 | 53 | 59 | 50 | 50 | 50 | 49 |

*Notes:*

(1) Le dépôt de l'adatome de cuivre a été effectué à partir de chlorure cuivreux dans une autre cellule avant l'électrolyse en présence de glyoxal.

(2) Cet exemple a été réalisé avec un anolyte contenant de l'acide chlorhydrique 1,1 N et du glyoxal 1,71 M.

(3) Le dépot de l'adatome de bismuth a été effectué à partir de l'oxyde de bismuth III dans une autre cellule avant l'électrolyse en présence de glyoxal.

Dans tous les autres exemples, l'adatome a été déposé sur l'anode platine.

Ce tableau montre qu'en appliquant l'invention avec des adatomes différents, on obtient de l'acide glyoxylique pauvre en glyoxal résiduel comparativement au procédé conduit dans des conditions analogues sans dopage de l'anode.

TABLEAU II - EXEMPLES 14 à 16

*Conditions opératoires:*
I = 1 $A/dm^2$
Catholyte = NaOH 1N
Anolyte = HCl 1,1 N
glyoxal 0,860 M

| | Exemple 14 | Exemple 15 | Exemple 16 |
|---|---|---|---|
| Anode | Platine | Platine | Platine |
| Adatome | Néant | Néant | $Ag^+$ (1) |

TABLEAU II - EXEMPLES 14 à 16 (Suite)

| | Exemple 14 | Exemple 15 | Exemple 16 |
|---|---|---|---|
| Température °C | 20 | 50 | 50 |
| V volts | 3 | 2,8 | 2,8 |
| Q/Qth | 1,22 | 1,22 | 1,22 |
| *Résultats:* | | | |
| Glyoxal résiduel | 146 | 77,5 | 17,2 |
| Acide glxoylique | 432 | 676 | 676 |
| Acide oxalique | 33 | 68 | 64 |
| Rendement chimique | 60,5% | 86,4% | 80,2% |
| Rendement électrique | 41% | 64% | 64% |

(1) Le dopage de l'anode a été réalisé en introduisant au démarrage de l'électrolyse 14 mg de nitrate d'argent dans le compartiment anodique.

A l'examen de ce tableau, il ressort qu'en appliquant la variante de l'invention, on obtient également un catholyte contenant des ions $OH^-$, un acide glyoxylique pauvre en glyoxal résiduel.

Les exemples suivants illustrent une autre variante de l'invention dans laquelle on utilise un électrolyseur équipé de deux compartiments séparés par une membrane anionique du commerce; ces deux compartiments ont un volume de 25 $cm^3$ environ et ils sont munis respectivement d'une anode et d'une cathode en platine, d'un moyen d'agitation magnétique et d'un réfrigérant pour éviter l'évaporation de l'eau lorsqu'on réalise les essais à une température supérieure à 20°C.

Les résultats obtenus figurent au tableau III ci-après:

TABLEAU III: Exemples 17-20

Influence des adatomes déposés sur l'anode et d'une membrane échangeuse d'anions

| exemples | 17 | 18 | 19 | 20 |
|---|---|---|---|---|
| *Conditions opératoires* | | | | |
| Electrodes | | platine | | |
| Electrolyte | | HCl 1,1 N | | |
| Intensité I A/$dm^2$ | | 1 | | |
| Voltage V | | 2,5 - 2,8 | | |
| Volume initial $cm^3$ | | 20 | | |
| Volume final $cm^3$ | | 21 - 22 | | |
| Membrane anionique | | RAIPORE 5035 L | | |
| Adatome | néant | $Sn^{++}$ | $Ag^+$ | |
| nature | | $SnCl_2$ | $AgNO_3$ | |
| quantité mmol/l | | 1,11 | 1,18 | |
| Température °C | 20 | 20 | 20 | 50 |
| Q/Qth | 1,20 | 1,20 | 1,21 | 1,21 |
| Quantité d'électricité C | 4790 | 4150 | 4180 | 4180 |
| Glyoxal initial mmol/l | 1035 | 895 | 895 | 895 |
| *Résultats* | | | | |
| Glyoxal résiduel mmol/l | 145 | 60,5 | 57 | 17 |
| Acide glyoxylique mmol/l | 650 | 575 | 585 | 629 |
| Acide oxalique mmol/l | 57 | 72 | 71 | 79 |
| Chlorures finals | 1,1N | | | |
| *Rendements* | | | | |
| Chimiques % | 73 | 69 | 70 | 72 |
| Electrique % | 53 | 53 | 54 | 58 |

A l'examen de ce tableau, on constate que l'emploi simultané d'une membrane anionique et d'une anode dopée conduit à des rendements chimiques et électriques supérieurs à ceux acquis avec le séparateur en verre fritté. En outre, la comparaison de l'exemple 17 avec les exemples 18-20 montre l'influence favorable de l'emploi d'une anode dopée sur les taux de glyoxal résiduels.

**Revendications**

1. Procédé de préparation d'acide glyoxylique par oxydation électrochimique anodique de glyoxal en solution aqueuse, sous agitation, à une température comprise entre 0°C et 70°C, dans un électrolyseur comportant, au moins un compartiment cathodique contenant une cathode et un catholyte, au moins un compartiment anodique contenant une anode et un anolyte constitué par une solution aqueuse de glyoxal et un électrolyte et entre ces deux compartiments au moins un séparateur, ledit procédé étant caractérisé en ce que l'anode est dopée par des adatomes métalliques choisis parmi l'argent, le bismuth, le cuivre, l'étain, le thallium.

2. Procédé selon la revendication 1, caractérisé par le fait que le séparateur est en verre fritté.

3. Procédé selon la revendication 1, caractérisé par le fait que le séparateur est une membrane échangeuse d'anions.

4. Procédé selon l'une des revendications 1 à 3, caractérisé par le fait que le catholyte est une solution aqueuse d'un hydroxyde de métal alcalin.

5. Procédé selon l'une quelconque des revendications 1 à 4, caractérisé per le fait que l'anode est dopée avec de l'étain.

6. Procédé selon l'une quelconque des revendications 1 à 4, caractérisé par le fait que l'anode est dopée avec de l'argent.

7. Procédé selon l'une quelconque des revendications 1 à 6, caractérisé par le fait que l'anolyte est une solution de glyoxal dans de l'acide chlorhydrique 1 ± 0,3 N.

8. Procédé selon l'une quelconque des revendications 1 à 7, caractérisé par le fait que le catholyte est une solution normale d'hydroxyde de sodium.

## Patentansprüche

1. Verfahren zur Herstellung von Glyoxalsäure durch elektrochemische anodische Oxidation von Glyoxal in wässriger Lösung unter Rühren bei einer Temperatur von 0 bis 70°C in einer Elektrolysevorrichtung, die mindestens ein Kathodenabteil mit einer Kathode und einem Katholyten, mindestens ein Anodenabteil mit einer Anode und einem Anolythen, bestehend aus einer wässerigen Lösung von Glyoxal und einem Elektrolyten, und zwischen den beiden Abteilen mindestens einen Separator umfasst, dadurch gekennzeichnet, dass die Anode durch Metallatome ausgewählt unter Silber, Wismut, Kupfer, Zinn und Thallium dotiert ist.

2. Verfahren nach Anspruch 1, dadurch gekennzeichnet, dass der Separator eine Glasfritte ist.

3. Verfahren nach Anspruch 1, dadurch gekennzeichnet, dass der Separator eine Anionenaustauschermembran ist.

4. Verfahren nach einem der Ansprüche 1 bis 3, dadurch gekennzeichnet, dass der Katholyt eine wässerige Lösung eines Alkalimetallhydroxids ist.

5. Verfahren nach einem der Ansprüche 1 bis 4, dadurch gekennzeichnet, dass die Anode mit Zinn dotiert ist.

6. Verfahren nach einem der Ansprüche 1 bis 4, dadurch gekennzeichnet, dass die Anode mit Silber dotiert ist.

7. Verfahren nach einem der Ansprüche 1 bis 6, dadurch gekennzeichnet, dass der Anolyt eine Lösung von Glyoxal in 1 ± 0,3 n Salzsäure ist.

8. Verfahren nach einem der vorhergehenden Ansprüche, dadurch gekennzeichnet, dass der Katholyt eine n Natriumhydroxidlösung ist.

## Claims

1. Process for producing glyoxylic acid by electrochemical anodic oxidation of glyoxal in aqueous solution, with stirring, at a temperature between 0°C and 70°C, in an electrolyser comprising at least one cathodic compartment containing a cathode and a catholyte, at least one anodic compartment containing an anode and an anolyte consisting of an aqueous solution of glyoxal and an electrolyte, and at least one separator between these two compartments, the said process being characterized in that the anode is doped with metallic adatoms chosen from silver, bismuth, copper, tin and thallium.

2. Process according to Claim 1, characterized in that the separator is made of fritted glass.

3. Process according to Claim 1, characterized in that the separator is an anion exchange membrane.

4. Process according to one of Claims 1 to 3, characterized in that the catholyte is an aqueous solution of an alkaline metal hydroxide.

5. Process according to any of Claims 1 to 4, characterized in that the anode is doped with tin.

6. Process according to any one of Claims 1 to 4, characterized in that the anode is doped with silver.

7. Process according to any one of Claims 1 to 6, characterized in that the anolyte is a solution of glyoxal in 1 ± 0.3 N hydrochloric acid.

8. Process according to any one of Claims 1 to 7, characterized in that the catholyte is a 1 N solution of sodium hydroxide.